# FASCICULE DE BREVET EUROPEEN

(11) **EP 2 756 851 B1**
(45) Date de publication et mention de la délivrance du brevet: **20.04.2016**
(21) Numéro de dépôt: 14151604.7
(22) Date de dépôt: 17.01.2014
(51) Int. Cl.: A61K 47/36, A23L 29/00

(54) **Produit d'assistance a l'ingestion d'un compose solide**
Hilfsprodukt zum Schlucken einer festen Zusammensetzung
Product to assist with the ingestion of a solid compound

(30) Priorité: 21.01.2013 FR 1300120
(43) Date de publication de la demande: 23.07.2014
(73) Titulaire: Eredova, 37000 Tours (FR)
(72) Inventeur: Bachelard-Chalmont, Eva, 37000 TOURS (FR)
(74) Mandataire: Thomas, Nadine

(56) Documents cités:
- FR-A1- 2 968 998
- US-E1- R E39 125
- Anonymous: "Pill Glide swallowing spray", Pill Glide , 2009, pages 1-2, XP002698309, Extrait de l'Internet: URL:http://www.pillglide.co.uk/product.asp [extrait le 2013-06-06]
- SHELLEY DIAMOND AND DANIELLE C LAVALLEE: "Experience With a Pill-Swallowing Enhancement Aid", CLINICAL PEDIATRICS, vol. 49, no. 4, 1 avril 2010 (2010-04-01), pages 391-393, XP009169686, CORTLANDT GROUP, OSSIGING, NY, US ISSN: 0009-9228

## Description

L'invention concerne un produit d'assistance à l'ingestion d'un composé solide individualisé à prendre par voie orale sous forme solide, tel qu'un médicament ou un complément alimentaire se présentant sous forme solide notamment d'un comprimé ou d'une gélule et similaire.

Les médicaments ou les compléments alimentaires solides qui se prennent à l'état solide par voie orale sont typiquement placés dans la bouche puis avalés en prenant une quantité d'eau ou autre boisson dans la bouche.

Toutefois certaines personnes éprouvent une difficulté pour avaler ces composés. Elles ressentent une sensation de gêne au moment de la prise du composé et manifestent un blocage d'origine psychologique et/ou physiologique, car le composé ne passe pas par le bol alimentaire et qu'il doit être avalé sans mastication. Elles sont gênées par le contact du composé dans leur bouche et peuvent avoir peur que le composé reste collé dans leur bouche et/ou la gorge ou passe de travers et qu'elles s'étouffent.

On a proposé par exemple dans la demande de brevet EP 1 385 552 une composition relativement visqueuse dans laquelle le composé solide à avaler est inséré juste avant sa prise puis l'ensemble composé/composition est mis dans la bouche de l'individu qui doit avaler ledit composé. La composition permet d'envelopper le composé à avaler et d'augmenter la poussée de ce composé dans la bouche et la gorge de l'individu concerné pour faciliter son ingestion. Toutefois cette technique peut entraîner un début d'altération du composé à prendre dans la bouche, donc avant son ingestion, ce qui peut être préjudiciable. De plus, à chaque prise de composé, il est nécessaire de prendre de cette composition pour ingérer ledit composé.

L'art antérieur comprend aussi "Pill Glide swallowing spray", Pill Glide, 2009.

L'invention vise à proposer un produit pour faciliter la prise orale d'un composé solide individualisé à avaler, qui soit facile d'emploi et n'altère pas le composé dans la bouche de l'individu qui doit prendre ce composé. Elle vise aussi à proposer un produit qui permette la prise de plusieurs composés à la suite les uns des autres, sans avoir à utiliser plusieurs prises de cette composition.

L'invention propose d'avoir recours à cette fin à un produit conçu pour tapisser les parois de la cavité buccale d'un individu en formant un film sur les parois de la cavité buccale et en assurant un effet de lubrification à l'égard du composé solide à avaler. Le produit selon l'invention vient en assistance à la déglutition d'un composé solide à avaler, en permettant de former un film lubrifiant sur les parois de la cavité buccale pour favoriser le glissement dudit composé à avaler et diminuer le ressenti de la présence du composé dans la cavité buccale.

L'invention fournit ainsi un produit d'assistance à l'ingestion de composés solides individualisés à administrer par voie orale sous forme solide, ledit produit comportant un liquide aqueux présenté sous forme à diffuser dans la cavité buccale, sous forme pulvérisable, et apte à former un film sur les parois de la cavité buccale et ayant un effet lubrifiant à l'égard des composés solides individualisés qui n'adhèrent pas à ce film. La composition dudit liquide comprend de la pectine en tant qu'agent filmogène et un agent lubrifiant.

Plus précisément, ladite composition filmogène comporte, pour 100 parts en poids de composition aqueuse, de la pectine en une quantité comprise entre 0.1 à 0.3 parts en poids.

Préférentiellement, l'agent lubrifiant comprend de la glycérine et/ou du sorbitol.

On entend par « un agent lubrifiant », qu'il y a un seul agent ou plusieurs agents.

On entend par « ingestion de composés solides individualisés », l'ingestion en général de tels composés, notamment de type comprimé ou gélules. Il peut s'agir de l'ingestion d'un seul composé ou de plusieurs composés les uns à la suite des autres et/ou voire en même temps.

Quand on parle ici des parois de la cavité buccale, on entend y comprendre les parois de la langue, du palais, du pharynx, de la gorge, pour ce qui est accessible par projection, notamment par pulvérisation, du liquide aqueux depuis l'extérieur de la bouche.

Le liquide comprend de préférence au moins 80 parts en poids d'eau pour 100 parts en poids de ladite composition aqueuse.

La formulation de la composition du liquide selon l'invention a été sélectionnée au terme de nombreux essais.

Conformément à l'invention, la pectine a été sélectionnée parmi les très nombreux polysaccharides qui sont connus comme agents épaississants pour solutions aqueuses, et qui sont comestibles. Selon l'invention, il a été montré que la pectine a un effet filmogène qui permet au liquide de former un film, après application, sur les parois de la cavité buccale et d'y subsister le temps de remplir son rôle de lubrifiant vis-à-vis des composés à avaler. De ce fait le liquide ayant une telle composition selon l'invention permet une bonne déglutition. Ceci permet aussi des prises successives de composés solides sans avoir à renouveler l'opération d'application, en particulier par pulvérisation du liquide dans la cavité buccale entre chaque ingestion de composé réalisée en prenant une gorgée d'eau avec. De plus grâce au caractère filmogène du liquide appliqué conféré par la pectine dans les conditions de l'invention, la sensation de contact avec le composé solide placé dans la cavité buccale est sensiblement réduite, ce qui diminue la gêne ressentie par l'individu lors du contact du solide à avaler sur les parois buccales. La composition du liquide selon l'invention va donc supprimer la sensation de gêne physiologique engendrée chez l'individu prenant le composé et par voie de conséquence enlever l'appréhension et la peur d'ordre psychologique à déglutir et/ou de plus l'aider physiologiquement à déglutir grâce au caractère film lubrifiant.

De préférence selon l'invention, la pectine est présente en une quantité inférieure à 0,5 part en poids pour 100 parts en poids de composition aqueuse du produit, de préférence encore inférieure à 0,3 part, pour que le liquide ne soit pas trop gélifié pour être apte à être facilement pulvérisé. Le jet pulvérisé n'étant pas trop directionnel, on évite ainsi une irritation du fond de la cavité buccale, et de plus le liquide se répartit bien dans la cavité buccale.

De préférence aussi la quantité en pectine est supérieure à 0,1 part en poids pour 100 parts en poids de composition aqueuse, pour que le liquide ne soit pas trop fluide.

Avantageusement selon l'invention, la quantité en pectine est de préférence comprise entre 0,1 et 0,3 part en poids pour 100 parts en poids de la composition aqueuse. De préférence elle est strictement comprise entre ces valeurs, c'est-à-dire que la part en pectine est de préférence strictement supérieure à 0,1 part et strictement inférieure à 0,3 part.

De préférence encore, la quantité en pectine est comprise entre 0,13 et 0,2 part en poids environ, pour obtenir les propriétés combinées de caractère filmogène, de pulvérisabilité et de viscosité qui sont souhaitables pour le liquide à appliquer suivant l'invention, notamment quand le liquide est appliqué par pulvérisation. Plus préférentiellement encore, la quantité en pectine est de 0,15 part en poids, environ, pour 100 parts en poids de la composition aqueuse.

Comme agents lubrifiants, l'invention prévoit d'utiliser de préférence le glycérol et/ou le sorbitol. Le glycérol est le propane 1, 2, 3 triol ; il est plus communément appelé glycérine. De préférence selon l'invention, la composition du liquide comprend du sorbitol en une quantité comprise entre 4 et 7 parts en poids pour 100 parts en poids de la composition aqueuse. Le sorbitol peut avoir un impact sur la viscosité finale de la composition aqueuse et il possède également un certain pouvoir édulcorant. De préférence, le sorbitol est présent en une quantité de l'ordre de 5 parts en poids pour 100 parts en poids de la composition aqueuse afin d'obtenir une viscosité du liquide adaptée à être projeté, notamment pulvérisable. Par ailleurs le liquide à appliquer dans ce cas n'a pas un goût sucré trop prononcé.

Selon l'invention, on utilise de préférence la glycérine en une quantité comprise entre 4 et 7 parts en poids pour 100 parts en poids de la composition aqueuse, de préférence encore en une quantité d'environ 5 parts en poids pour 100 parts en poids de la composition aqueuse.

Le sorbitol et la glycérine sont de préférence utilisés ensemble aux proportions qui ont été indiquées précédemment.

De préférence, le liquide à appliquer a une composition comportant en outre des agents additifs de qualité alimentaire, choisis parmi les agents conservateurs, les agents édulcorants, les agents aromatisants, et leurs mélanges.

Pour le confort de l'utilisateur, le liquide à appliquer est tel qu'il présente un goût sucré et de préférence aussi aromatisé pour le rendre plus agréable à prendre.

En plus du sorbitol qui a été retenu comme l'un des constituants lubrifiants préférés pour composer le liquide, on peut ajouter un ingrédient présentant un plus fort pouvoir sucrant. Cet ingrédient est de préférence le sucralose. Afin d'avoir un goût sucré ressenti comme peu ou pas trop écoeurant, on utilise de préférence moins de 0,02 part en poids de sucralose pour 100 parts en poids de la composition aqueuse.

En ce qui concerne les agents aromatisants, on peut notamment utiliser les arômes alimentaires comme ceux donnant un goût de menthe ou un goût fruité.

Le liquide à appliquer étant formulé en milieu aqueux, sa composition comporte avantageusement un agent conservateur afin d'éviter toute prolifération bactériologique lors de son utilisation. De préférence l'agent conservateur est le benzoate de sodium, car il est l'un des plus simples à mettre en oeuvre et l'un des plus efficaces. Il est préférentiellement utilisé en une quantité comprise entre 0,1 et 1 part en poids pour 100 parts en poids de la composition aqueuse.

C'est en milieu acide, dans une gamme de pH allant de 4,7 à 5,1, que le benzoate de sodium est le plus efficace. La composition du liquide selon l'invention comporte donc en outre et si besoin un ajusteur de pH en quantité suffisante pour obtenir un pH de la composition compris entre 4,7 et 5,1. De préférence selon l'invention, l'ajusteur de pH est l'acide citrique.

De préférence selon l'invention, on sélectionne comme composition pour réaliser le liquide que comprend le produit d'assistance à l'ingestion d'un composé solide individualisé à avaler, notamment tel qu'un médicament ou complément alimentaire du type comprimé ou gélule, une composition qui est essentiellement constituée, pour 1 parts en poids de composition aqueuse :
- de 4 à 6 parts en poids de glycérine,
- de 4 à 6 parts en poids de sorbitol,
- d'environ 0,15 part en poids de pectine,
- de 0,1 à 1 part en poids de benzoate de sodium
- de l'acide citrique en proportion propre à ajuster le pH de ladite composition à une valeur comprise entre 4,7 et 5,1,
- de 0 à 0,02 part en poids de sucralose.
- de 0 à 2 parts en poids d'un agent aromatisant,
et pour le reste d'au moins 85 parts en poids d'eau, les proportions précises des divers constituants, eau comprise, étant choisies telles que leur somme soit égale à 100 parties en poids.

Le produit selon l'invention permet de répondre à l'ensemble des critères que sont l'efficacité dans un contexte d'aide à la déglutition, la facilité d'utilisation, la commodité pour ingérer plusieurs composés solides les uns à la suite des autres sans avoir à renouveler l'opération d'application, en particulier de pulvérisation, du liquide dans la cavité buccale de l'individu concerné.

De plus le liquide ainsi composé revêt un aspect agréable d'utilisation.

Selon l'invention, le liquide à appliquer est conditionné dans un matériel permettant de le diffuser, notamment de le projeter dans la cavité buccale. Plus particulièrement le produit selon l'invention est conçu pour se présenter de préférence sous forme d'un réservoir muni d'une tête de projection, de préférence une tête de pulvérisation, ledit réservoir contenant le liquide à appliquer dans la cavité buccale, et dont la composition en milieu aqueux a été décrite précédemment. Par exemple le produit se présente sous forme d'un flacon muni d'ne pompe distributrice sans gaz propulseur ou d'un flacon muni d'une pompe aérosol. Selon une autre variante de l'invention, le produit peut aussi se présenter sous forme d'une pipette contenant le liquide qui a été décrit.

L'invention concerne aussi un procédé pour faciliter la déglutition. C'est un procédé d'assistance à l'ingestion de composés solides individualisés, notamment du type comprimé ou gélule, administrés à l'état solide par voie orale, consistant, préalablement à l'ingestion des composés solides individualisés, à tapisser la cavité buccale, de l'individu à qui vont être administrés les composés solides, au moyen d'un liquide en milieu aqueux apte à former un film sur les parois de la cavité buccale et ayant un effet lubrifiant à l'égard des composés à ingérer qui n'adhèrent pas à ce film, à l'aide d'un matériel permettant de diffuser, en particulier de projeter, ledit liquide La composition dudit liquide comprend de la pectine en tant qu'agent filmogène et un agent lubrifiant.

L'ingestion du composé est faite de préférence en buvant une gorgée d'une boisson après application du liquide sur les parois de la cavité buccale et une fois le composé à ingérer mis dans la cavité buccale, pour aider à pousser le composé à avaler vers l'oesophage.

Selon l'invention, le liquide à appliquer se présente sous une forme pulvérisable.

De préférence selon le procédé de l'invention, on utilise un produit tel que décrit précédemment.

Selon le procédé de l'invention, on pulvérise de préférence sur les parois buccales, un liquide en milieu aqueux dont la composition est constituée essentiellement, pour 100 parts en poids de ladite composition aqueuse,
- de 4 à 6 parts en poids de glycérine,
- de 4 à 6 parts en poids de sorbitol,
- d'environ 0,15 part en poids de pectine,
- de 0,1 à 1 part en poids de benzoate de sodium
- d'acide citrique en proportion propre à ajuster le pH de ladite composition à une valeur comprise entre 4,7 et 5,1,
- de 0 à 0,02 part en poids de sucralose.
- de 0 à 2 parts en poids d'un agent aromatisant,
et pour le reste d'au moins 85 parts en poids d'eau.

Les proportions précises des divers constituants, eau comprise, sont donc choisies telles que leur somme soit égale à 100 parts en poids.

Selon le procédé de l'invention, plusieurs composés individualisés, au moins deux composés, peuvent être ingérés successivement par un individu, après une seule opération d'application du liquide, de préférence par pulvérisation, du liquide décrit, dans la cavité buccale de l'individu pour prendre le premier composé.

L'invention sera mieux comprise à l'aide des exemples suivants.

### EXEMPLES:

### Exemple 1 selon l'invention :

On réalise un produit selon l'invention en préparant un liquide aqueux de la manière qui suit, et dont la composition est décrite pour obtenir 100 g de liquide aqueux.

On dissout 0,15 g de pectine dans 5 g de glycérine. La pectine est une pectine extraite de la peau de citron, de qualité alimentaire. Elle a un degré d'estérification élevé ; son degré d'estérification est compris entre 65 et 75 %. Dans cet exemple la pectine est fournie par la société CP Kelco.

Ensuite à 85 g d'eau purifiée, on ajoute sous agitation le mélange de glycérine et de pectine, puis 5 g de sorbitol.

On ajoute ensuite 0,01 g de sucralose.

On ajoute 0,9g d'arôme de menthe

On introduit comme agent conservateur, 0,2 g de benzoate de sodium

On ajoute ensuite la quantité nécessaire d'acide citrique pour que la composition aqueuse finale présente un pH compris entre 4,7 et 5,1, afin de rendre optimale l'efficacité du benzoate de sodium.

Pour terminer, on complète avec de l'eau pour avoir au total 100g de liquide, eau comprise, tout en vérifiant que le pH reste dans la plage de 4,7 à 5,1.

Le liquide obtenu est mis dans le réservoir d'un flacon pulvérisateur ayant des dimensions adaptées à ce que ce flacon tienne facilement dans la main, par exemple un petit flacon de moins de 50 ml, muni d'une pompe distributrice sans gaz propulseur, dite "pompe spray". Le liquide est parfaitement pulvérisable.

Le caractère filmogène et lubrifiant du liquide obtenu a été vérifié. Le produit a été testé sur quelques individus ayant une appréhension pour avaler un composé de type comprimé ou gélule.

Dans chaque cas, on pulvérise le liquide obtenu dans la cavité buccale de l'individu. Un comprimé est ensuite placé sur la langue comme usuellement et un verre d'eau est bu immédiatement pour aider à la déglutition dudit comprimé.

Ces individus disent ne pas sentir de manière prononcée le contact du comprimé lorsque ce dernier est placé dans leur cavité buccale. Le comprimé ne colle pas dans la bouche, il est facilement et vite dégluti pour être ingéré. De plus le liquide appliqué dans la bouche a un goût légèrement sucré ressenti comme agréable et non écoeurant.

On vérifie également qu'une seule opération de pulvérisation permet d'avaler plusieurs comprimés, dans cet exemple trois comprimés, l'un à la suite de l'autre, sans avoir besoin de repulvériser du liquide entre chaque comprimé. L'opération de pulvérisation avant la prise du premier composé solide peut néanmoins comporter l'envoi de plusieurs jets de liquide, par exemple 2 à 3, dans la cavité buccale.

### Exemples comparatifs 2 à 3 :

Les exemples 2 et 3 sont des exemples comparatifs montrant que la gomme de xanthane qui est un polysacharide comestible autre que la pectine, ne permet pas d'obtenir l'effet filmogène comme obtenu avec la pectine.

Ils sont réalisés selon l'exemple 1 dans lequel la pectine a été remplacée par la gomme de xanthane selon les quantités indiquées dans le tableau ci-inclus.

### Exemples 4 à 5 :

Les exemples 4 et 5 sont réalisés selon l'exemple 1 mais la pectine est mise selon les quantités indiquées dans le tableau suivant.

L'exemple 4 montre qu'il est préférable que la quantité en pectine, utilisée dans les conditions de l'exemple 1, soit strictement supérieure à 0,1 g pour 100 g de composition afin d'avoir un liquide aqueux pas trop fluide.

L'exemple 5 montre qu'il est préférable que la quantité en pectine, utilisée dans les conditions de l'exemple 1, soit strictement inférieure à 0,3 g pour obtenir un liquide facilement pulvérisable.

| Exemples | Gomme de xanthane (g) | Pectine (g) | Consistance du liquide | Effet filmogène | Pulvérisabilité du liquide |
|---|---|---|---|---|---|
| Exemple 1 | 0 | 0,15 | Bonne viscosité | OUI | Bonne |
| Exemple 2 comparatif | 0,1 | 0 | Trop liquide | NON | Bonne |
| Exemple 3 comparatif | 0,15 | 0 | Bonne viscosité | NON | Bonne |
| Exemple 4 | 0 | 0,1 | Trop fluide | OUI | Bonne |
| Exemple 5 | 0 | 0,3 | Trop gélifiée | OUI | Mauvaise |

On a montré par ailleurs qu'il est préférable que le liquide, comme réalisé selon l'exemple 1, ait une composition qui comporte plus de 3 parts en poids de sorbitol pour avoir un effet lubrifiant suffisant. La quantité d'environ 5 parts est préférable, une quantité supérieure à 7 parts est possible mais n'apporte pas d'effet supplémentaire.

En conclusion, le produit selon l'invention comporte un liquide qui permet d'améliorer le processus physiologique de déglutition d'un composé solide individualisé à avaler sous forme solide, en créant un film lubrifiant dans la cavité buccale. Ce liquide permet de faciliter la déglutition du composé solide grâce à son effet lubrifiant vis-à-vis du composé à avaler, et de réduire la sensation du contact de ce composé solide sur les surfaces internes de la cavité buccale grâce à son effet « filmogène ». Le produit selon l'invention permet donc aux individus qui ressentent une gêne psychologique ou physiologique pour avaler leur médicament ou complément alimentaire ou autres composés solides similaires notamment tels que comprimés ou gélules, de ne plus avoir la sensation que ledit composé reste collé dans la bouche.

Le produit selon l'invention permet de répondre à l'ensemble des critères que sont l'efficacité dans un contexte d'aide à la déglutition, la facilité d'utilisation et l'aspect agréable d'utilisation du liquide qu'il comporte ainsi que la prise de plusieurs composés les uns à la suite des autres sans avoir à renouveler l'opération d'application du liquide dans la bouche de l'individu concerné.

La description qui précède explique clairement comment l'invention permet d'atteindre les objectifs qu'elle s'est fixés. En particulier elle peut s'appliquer à tout composé à avaler sous forme solide similaire à un médicament ou un complément alimentaire se présentant sous forme solide notamment d'un comprimé ou d'une gélule, comme par exemple les capsules molles qui sont formées d'ingrédients plus ou moins liquides encapsulés dans une enveloppe souple soudée.

## Revendications

1. Produit d'assistance à l'ingestion de composés solides individualisés à administrer par voie orale sous forme solide, ledit produit comportant un liquide aqueux présenté sous forme à diffuser dans la cavité buccale sous forme pulvérisable, apte à former un film sur les parois de la cavité buccale et ayant un effet lubrifiant à l'égard des composés solides individualisés, et dont la composition comprend - de la pectine en tant qu'agent filmogène, ladite composition filmogène comportant, pour 100 parts en poids de composition aqueuse, de la pectine en une quantité comprise entre 0.1 à 0.3 parts en poids ; -du sorbitol en tant qu'agent lubrifiant, en une quantité comprise entre 4 et 7 parts en poids pour 100 parts en poids de composition aqueuse, et/ou de la glycérine en tant qu'agent lubrifiant, en une quantité comprise entre 4 et 7 parts en poids pour 100 parts en poids de composition aqueuse.

2. Produit selon la revendication précédente, **caractérisé en ce que** ledit liquide comprend au moins 80 parts en poids d'eau pour 100 parts en poids de composition aqueuse.

3. Produit selon l'une des revendications 1 ou 2, **caractérisé en ce que** la composition comporte de la pectine en une quantité comprise entre 0,13 et 0,2 part en poids, de préférence encore en une quantité égale à environ 0,15 part en poids.

4. Produit selon l'une des revendications précédentes, **caractérisé en ce que** la composition comporte du sorbitol en une quantité de préférence égale à environ 5 parts en poids et/ou de la glycérine en une quantité de préférence égale à environ 5 parts en poids.

5. Produit selon l'une des revendications précédentes, **caractérisé en ce que** la composition comporte des agents additifs compatibles, choisis parmi les agents conservateurs, les agents édulcorants, les agents aromatisants et leurs mélanges.

6. Produit selon la revendication précédente, **caractérisé en ce que** la composition comporte un agent édulcorant, en particulier le sucralose, et de préférence en une quantité d'au plus égale à 0,02 part en poids pour 100 parts en poids de la composition aqueuse.

7. Produit selon l'une des revendications 5 ou 6, **caractérisé en ce que** la composition comporte comme agent conservateur du benzoate de sodium, de préférence en une quantité d'au plus égale à 1 part en poids pour 100 parts en poids de la composition aqueuse, et un ajusteur de pH en quantité suffisante pour obtenir un pH de ladite composition compris entre 4,7 et 5,1, ledit ajusteur de pH étant de préférence l'acide citrique.

8. Produit selon la revendication 1, **caractérisé en ce que** le liquide a une composition constituée :
- de 4 à 6 parts en poids de glycérine,
- de 4 à 6 parts en poids'de sorbitol,
- d'environ 0,15 part en poids de pectine,
- de 0,1 à 1 part en poids de benzoate de sodium
- d'acide citrique en quantité permettant d'ajuster le pH de ladite composition entre 4,7 et 5,1,
- de 0 à 0,02 part en poids de sucralose.
- de 4 à 2 parts en poids d'un agent aromatisant,
et pour le reste d'au moins 85 parts en poids d'eau, les proportions précises des divers constituants, y compris d'eau, étant choisies telles que leur somme soit égale à 100 parts en poids.

9. Produit selon l'une des revendications précédentes, **caractérisé en ce qu'**il comporte un réservoir qui contient ledit liquide aqueux et qui est muni d'un moyen de projection dudit liquide, de préférence d'une tête de pulvérisation,

10. Procédé d'assistance à l'ingestion de composés solides individualisés, administrés à l'état solide par voie orale, consistant, préalablement à l'ingestion des composés solides individualisés, à tapisser la cavité buccale à l'aide d'un matériel permettant de diffuser, sous forme pulvérisable ledit liquide, et au moyen d'un liquide en milieu aqueux apte à former un film sur les parois de la cavité buccale et ayant un effet lubrifiant à l'égard des composés à ingérer et dont la composition comporte de la pectine en tant qu'agent filmogène et un agent lubrifiant, ladite composition filmogène comportant, pour 100 parts en poids de composition aqueuse, de la pectine en une quantité comprise entre 0.1 à 0.3 parts en poids, et du sorbitol en tant qu'agent lubrifiant, en une quantité comprise entre 4 et 7 parts en poids pour 100 parts en poids de composition aqueuse, et/ou de la glycérine en tant qu'agent lubrifiant, en une quantité comprise entre 4 et 7 parts en poids pour 100 parts en poids de composition aqueuse.

11. Procédé selon la revendication précédente, **caractérisée en ce qu'**on utilise un produit tel que défini à l'une des revendications 1 à 9.

## Patentansprüche

1. Produkt als Schluckhilfe für individuell zusammengestellte Festkörper, die in fester Form einzunehmen sind, wobei das Produkt eine wässrige Flüssigkeit enthält, die in einer in der Mundhöhle pulverförmig verteilbaren Form präsentiert wird, und in der Lage ist, an den Wänden der Mundhöhle einen Film zu bilden, der eine gleitende Wirkung für die individuell zusammengestellten Festkörper hat, und dessen Zusammensetzung als filmbildendes Mittel Pektin enthält, wobei besagte filmbildende Zusammensetzung Pektin in einer Menge zwischen 0,1 und 0,3 Gewichtsteilen bezogen auf 100 Gewichtsteile der wässrigen Zusammensetzung, Sorbitol als Gleitmittel in einer Menge zwischen 4 und 7 Gewichtsteilen bezogen auf 100 Gewichtsteile der wässriger Zusammensetzung, und/oder Glyzerin als Gleitmittel in einer Menge zwischen 4 und 7 Gewichtsteilen bezogen auf 100 Gewichtsteile der wässrigen Zusammensetzung enthält.

2. Produkt nach dem vorhergehenden Anspruch, **dadurch gekennzeichnet, dass** besage Flüssigkeit mindestens 80 Gewichtsteile Wasser bezogen auf 100 Gewichtsteile wässriger Zusammensetzung enthält.

3. Produkt nach Patentanspruch 1 oder 2, **dadurch gekennzeichnet, dass** die Zusammensetzung Pektin in einer Menge zwischen 0,13 und 0,2 Gewichtsteilen, vorzugsweise aber in einer Menge von 0,15 Gewichtsteilen enthält.

4. Produkt nach einem der vorhergehenden Ansprüche, **dadurch gekennzeichnet, dass** die Zusammensetzung Sorbitol in einer Menge enthält, die vorzugsweise 5 Gewichtsteilen entspricht, und/oder Glyzerin in einer Menge, die vorzugsweise 5 Gewichtsteilen entspricht.

5. Produkt nach einem der vorhergehenden Ansprüche, **dadurch gekennzeichnet, dass** die Zusammensetzung kompatible Zusatzstoffe enthält, die aus Konservierungsstoffen, Süßmitteln, Geschmacksstoffen und deren Mischungen ausgewählt werden.

6. Produkt nach dem vorhergehenden Anspruch, **dadurch gekennzeichnet, dass** die Zusammensetzung ein Süßungsmittel, insbesondere aber Sucralose enthält, vorzugsweise in einer Menge von maximal 0,02 Gewichtsteilen bezogen auf 100 Gewichtsteile der wässrigen Zusammensetzung.

7. Produkt nach Patentanspruch 5 oder 6, **dadurch gekennzeichnet, dass** die Zusammensetzung das Konservierungsmittel Natriumbenzoat vorzugsweise in einer Menge von maximal 1 Gewichtsteil bezogen auf 100 Gewichtsteile der wässrigen Zusammensetzung enthält, und ein Mittel zum Einstellen des pH-Werts in einer Menge, die ausreicht, um bei der Zusammensetzung einen pH-Wert von 4,7 und 5,1 zu erzielen, wobei das Mittel zum Einstellen des pH-Werts vorzugweise Zitronensäure ist.

8. Produkt nach Anspruch 1, **dadurch gekennzeichnet, dass** die Flüssigkeit wie folgt zusammengesetzt ist:
- zu 4 bis 6 Gewichtsteilen aus Glyzerin,
- zu 4 bis 6 Gewichtsteilen aus Sorbitol,
- zu ungefähr 0,15 Gewichtsteilen aus Pektin,
- zu 0,1 bis 1 Gewichtsteil aus Natriumbenzoat
- Zitronensäure in einer Menge, die so bemessen ist, dass der pH-Wert besagter Zusammensetzung zwischen 4,7 und 5,1 liegt.
- 0 bis 0,02 Gewichtsteile Sucralose
- 0 bis 2 Gewichtsteile eines Geschmacksmittels,
und für den Rest 85 Gewichtsteile Wasser, wobei die genauen Anteile der einzelnen Bestandteile, einschließlich Wasser, so ausgewählt werden, dass die Summe 100 Gewichtsteile ergibt.

9. Produkt nach einem der vorhergehenden Ansprüche, **dadurch gekennzeichnet, dass** es einen Behälter umfasst, der besagte wässrige Flüssigkeit enthält, und mit einer Sprühvorrichtung für besagte Flüssigkeit, vorzugsweise einem Sprühkopf, versehen ist.

10. Verfahren zur leichteren Einnahme eines individuell zusammengestellten Festkörpers, der im festem Zustand oral eingenommen wird, und darin besteht, dass vor der Einnahme des individuell zusammengestellten Festkörpers die Mundhöhle mit einer Vorrichtung beschichtet wird, welche die sprühförmige Verteilung einer Flüssigkeit in wässriger Umgebung ermöglicht, und in der Lage ist, an den Wänden der Mundhöhe einen Film zu bilden, der einen Gleiteffekt in Bezug auf die zu schluckenden Festkörper hat, und dessen Zusammensetzung Pektin als filmbildendes Mittel sowie ein Gleitmittel umfasst, wobei besagtes filmbildendes Mittel zu 100 Gewichtsanteilen Pektin in einer Menge zwischen 0,1 und 0,3 Gewichtsteilen, sowie Sorbitol als Gleitmittel in einer Menge zwischen 4 und 7 Gewichtsteilen bezogen auf 100 Gewichtsanteile wässriger Lösung enthält, und/oder Glyzerin als Gleitmittel in einer Menge zwischen 4 und 7 Gewichtsteilen bezogen auf 100 Gewichtsteile wässrige Zusammensetzung.

11. Verfahren nach dem vorhergehenden Anspruch, **dadurch gekennzeichnet, dass** man ein Produkt gemäß den Ansprüchen 1 bis 9 benutzt.

## Claims

1. A product for facilitating the ingestion of individualized solid compounds to be orally administered in a solid form, said product comprising an aqueous liquid in a form able to diffuse into the buccal cavity, in a sprayable form, capable of forming a film on the walls of the buccal cavity and having a lubricating effect relative to the individualized solid compounds, and the composition of which comprises - pectin as a film-forming agent, said film-forming composition comprising, per 100 parts by weight of the aqueous composition, pectin in an amount ranging from 0.1 to 0.3 parts by weight; - sorbitol as the lubricating agent, in an amount ranging from 4 to 7 parts by weight per 100 parts by weight of the aqueous composition, and/or glycerin as the lubricating agent, in an amount ranging from 4 to 7 parts by weight per 100 parts by weight of the aqueous composition.

2. A product according to the preceding claim, **characterized in that** said liquid comprises at least 80 parts by weight of water per 100 parts by weight of the aqueous composition.

3. A product according to one of claims 1 or 2, **characterized in that** the composition comprises pectin in an amount ranging from 0.13 to 0.2 parts by weight, more preferably in an amount equal to about 0.15 parts by weight.

4. A product according to one of the preceding claims, **characterized in that** the composition comprises sorbitol in an amount preferably equal to about 5 parts by weight and/or glycerin in an amount preferably equal to about 5 parts by weight.

5. A product according to one of the preceding claims, **characterized in that** the composition contains compatible additives selected from the preservatives, the sweeteners, the flavoring agents and the mixtures thereof.

6. A product according to the preceding claim, **characterized in that** the composition contains a sweetener, more particularly sucralose, and preferably in an amount of no more than 0.02 part by weight per 100 parts by weight of the aqueous composition.

7. A product according to one of claims 5 or 6, **characterized in that** the composition contains sodium benzoate, as a preservative, preferably in an amount of no more than 1 part by weight per 100 parts by weight of the aqueous composition, and a pH adjuster in an amount sufficient to achieve a pH of said composition ranging from 4.7 to 5.1, with said pH adjuster preferably being citric acid.

8. A product according to claim 1, **characterized in that** the liquid has a composition consisting of:
- 4 to 6 parts by weight of glycerin,
- 4 to 6 parts by weight of sorbitol,
- about 0.15 part by weight of pectin,
- from 0.1 to 1 part by weight of sodium benzoate
- citric acid in an amount making it possible to adjust the pH of said composition between 4.7 and 5.1,
- from 0 to 0.02 parts by weight of sucralose,
- from 0 to 2 parts by weight of a flavoring agent,
and the rest being at least 85 parts by weight of water, with the precise proportions of the various components, including water, being chosen such that the sum thereof is equal to 100 parts by weight.

9. A product according to one of the preceding claims, **characterized in that** it comprises a tank which contains said aqueous liquid and which is provided with means for projecting said liquid, preferably a sprayhead.

10. A method for facilitating the ingestion of individualized solid compounds to be orally administered in a solid form, consisting, prior to the ingestion of the individualized solid compounds, in lining the buccal cavity using a device making it possible to diffuse said liquid in a sprayable form, and using a liquid in an aqueous medium capable of forming a film on the walls of the buccal cavity and having a lubricating effect relative to the compounds to be swallowed, and the composition of which comprises pectin as a film-forming agent and a lubricating agent, with said film-forming composition comprising, per 100 parts by weight of the aqueous composition, pectin in an amount ranging from 0.1 to 0.3 parts by weight; and sorbitol as the lubricating agent, in an amount ranging from 4 to 7 parts by weight per 100 parts by weight of the aqueous composition, and/or glycerin as the lubricating agent, in an amount ranging from 4 to 7 parts by weight per 100 parts by weight of the aqueous composition.

11. A method according to the preceding claim, **characterized in that** a product as defined in one of claims 1 to 9 is used.
